(19) Europäisches Patentamt
European Patent Office
Office européen des brevets

(11) Veröffentlichungsnummer: **0 217 269 B1**

(12) **EUROPÄISCHE PATENTSCHRIFT**

(45) Veröffentlichungstag der Patentschrift: **01.08.90**

(51) Int. Cl.$^5$: **C 07 C 239/08**

(21) Anmeldenummer: **86113069.8**

(22) Anmeldetag: **23.09.86**

(54) **Verfahren zur Herstellung von N-Alkyl-substituierten Hydroxylammoniumchloriden.**

(30) Priorität: **04.10.85 DE 3535451**

(43) Veröffentlichungstag der Anmeldung:
**08.04.87 Patentblatt 87/15**

(45) Bekanntmachung des Hinweises auf die Patenterteilung:
**01.08.90 Patentblatt 90/31**

(84) Benannte Vertragsstaaten:
**AT BE CH DE FR GB IT LI**

(56) Entgegenhaltungen:

**THE JOURNAL OF THE AMERICAN CHEMICAL SOCIETY, Band 79, November 1957, Seiten 5739-5754; W.D. EMMONS "The preparation and properties of Oxaziranes"**

**CHEMICAL ABSTRACTS, Band 74, Nr. 25, 21. Juni 1971, Seite 482, Spalte 1, Zusammenfassungsnr. 140418v, Columbus, Ohio, US; B.C. CHALLIS et al.: "Hydrolysis of oxaziridines. I. Kinetics of 2-tertbutyloxaziridines in strong acids"**

(73) Patentinhaber: **BAYER AG**
**D-5090 Leverkusen 1 Bayerwerk (DE)**

(72) Erfinder: **Schalenbach, Rolf, Dr.**
**In der Kreuzau 5**
**D-5000 Köln 91 (DE)**
Erfinder: **Waldmann, Helmut, Dr.**
**Henry-T.-von-Böttinger-Strasse 15**
**D-5909 Leverkusen (DE)**
Erfinder: **Ingendoh, Axel, Dr.**
**Engstenberger Höhe 10**
**D-5068 Odenthal-Osenau (DE)**

(56) Entgegenhaltungen:
**J. MARCH "Advanced Organic Chemistry Reactions, Mechanisms and Structure", 1968, 2. Auflage, McGraw-Hill;**

**EP 0 217 269 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die vorliegende Erfindung betrifft ein Verfahren zur Herstellung von N-Alkyl-substituierten Hydroxylammoniumchloriden durch die Umsetzung bestimmter Arylaldimine mit einer Percarbonsäure und Hydrolyse des dabei gebildeten Oxaziridins.

Die bekannten N-Alkyl-substituierten Hydroxylamine sind wichtige Zwischenprodukte in der organischen Synthese (siehe z.B. R. S. Sander and W. Karo, "Organic Functional Group Preparations", Academic Press, 1972, 12/III, S. 353). Einige von ihnen weisen auch eine biologische Wirkung auf (siehe z.B. M. Tisler, Math. Naturw. R. *32*, (1983)). Beispielsweise inhibiert Hadacidin (= N-Formyl-N-hydroxyl-glycin) das Wachstum von Bakterien, Tumorzellen und tierischem und pflanzlichem Gewebe (siehe E. A. Kaczka, C. O. Gittermann, E. L. Delaney und K. Falkers, Biochemistry, *I*, 340 (1962)).

Es ist bekannt (siehe z.B. W. D. Emmons, J.A.C.S. *79*, 5739—5754 (1957)), daß man Arylaldimine, die eine unsubstituierte oder eine mit einer Nitrogruppe substituierte Phenylgruppe enthalten, mit Peressigsäure in das entsprechende Oxaziridin überführen, dieses durch Destillation isolieren, anschließend mit wäßrig-methanolischer Schwefelsäure hydrolysieren und durch Zugabe von Alkali das entsprechende freie, N-alkylierte Hydroxylamin erhalten kann. Der Einsatz von Salzsäure zur Hydrolyse derartiger Oxaziridine ist nicht möglich, da zwischen Chloridionen und derartigen Oxaziridinen eine Redoxreaktion stattfindet, wobei Chloridionen in Chlor und die Oxaziridine in Amine übergehen [siehe a.a.O., Fußnote (5) auf Seite 5741]. Außerdem wird die Peressigsäure in situ aus 90 %igem wäßrigem Wasserstoffperoxid hergestellt (siehe a.a.O., Seite 5734, linke Spalte). Die Handhabung derartig konzentrierter Wasserstoffperoxid-Lösungen ist insbesondere bei größeren Ansätzen sicherheitstechnisch außerordentlich problematisch.

Für die Verwendung von N-Alkyl-substituierten Hydroxylaminen als Zwischenprodukte und/oder biologische Wirkstoffe ist es von Vorteil, diese in einer Form bereitzuhalten, in der sie lagerstabil sind. Im allgemeinen sind die freien N-Alkyl-substituierten Hydroxylamine hinsichtlich ihrer Lagerstabilität nicht befriedigend. Im Gegensatz dazu weisen N-Alkyl-substituierte Hydroxylammoniumsalze im allgemeinen eine wesentlich bessere Lagerstabilität auf, jedoch kristallisieren diese, mit Ausnahme der Chloride, häufig nur schlecht oder gar nicht, was ihre Herstellung, Isolierung und Handhabung sehr erschwert.

Die Herstellung von N-Alkyl-substituierten Hydroxylammoniumchloriden über Oxaziridine, die eine unsubstituierte oder mit einer Nitrogruppe substituierte Phenylgruppe enthalten, ist, wie eingangs geschildert, bisher nicht möglich. Bekannte Herstellungsverfahren für N-Alkyl-substituierte Hydroxylammoniumchloride sind ebenfalls nachteilig. So liefert die Umsetzung von Hydroxylamin mit Alkylchloriden ein Gemisch aus freien Mono- und Di-alkylhydroxylaminen und Trialkylhydroxylammoniumchlorid (siehe Houben-Weyl, Band X/1, S. 1100, (1972)), und die an sich sehr selektive Reduktion von Oximen zu Hydroxylaminen mit $NaCNBH_3$ (siehe E. G. E. Jahngen und E. F. Rossomando, Synthetic Comm. *12*, 601 (1982)) ist wegen der hohen Kosten und schwierigen Zugänglichkeit zu größeren Mengen dieses Reduktionsmittels allenfalls für Umsetzungen im Labormaßstab geeignet.

Es besteht also immer noch ein dringendes Bedürfnis nach einem einfachen und wirtschaftlichen Verfahren zur Herstellung von N-Alkyl-substituierten Hydroxylammoniumchloriden.

Es wurde nun gefunden, daß man N-Alkyl-substituierte Hydroxylammoniumchloride herstellen kann, indem man ein Arylaldimin der Formel

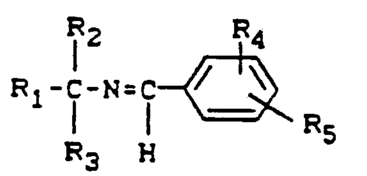

(I),

in der

$R_1$, $R_2$ und $R_3$ unabhängig voneinander für Wasserstoff, gegebenenfalls substituiertes gesättigtes Alkyl, gegebenenfalls substituiertes gesättigtes Cycloalkyl und/oder gegebenenfalls substituiertes Alkinyl stehen, wobei $R_1$ und $R_2$ gemeinsam mit dem C-Atom, an das sie gebunden sind, auch einen gegebenenfalls substituierten gesättigten Cycloalkylrest bilden können und

$R_4$ und $R_5$ unabhängig voneinander für Wasserstoff, gegebenenfalls substituiertes, gesättigtes Alkyl, gegebenenfalls substituiertes gesättigtes Cycloalkyl, gegebenenfalls substituiertes gesättigtes Alkoxy und/oder gegebenenfalls substituiertes gesättigtes Cycloalkoxy stehen, wobei mindestens einer der Reste $R_4$ und $R_5$ eine andere Bedeutung als Wasserstoff hat,

mit Perpropionsäure in Gegenwart eines Lösungsmittels umsetzt, dann zumindest Propionsäure abtrennt und anschließend eine Hydrolyse mit wäßriger Salzsäure durchführt.

Soweit $R_1$, $R_2$ und/oder $R_3$ für gegebenenfalls substituiertes gesättigtes Alkyl stehen kommen hierfür beispielsweise geradkettige oder verzweigte gesättigte Alkylreste mit insgesamt 1 bis 15 C-Atomen, davon 0 bis 3 sekundären C-Atomen und/oder 0 bis 3 tertiären C-Atomen in Frage. Als Substituenten an solchen

gesättigten Alkylresten kommen beispielsweise in Frage: gesättigte Cycloalkylreste, Arylreste, Alkinylreste und/oder Heteroatome, wie Halogen-, Sauerstoff-, Schwefel-, Stickstoff- und/oder Phosphoratome enthaltende Reste. Derartige gesättigte Cycloalkylreste können z.B. 3 bis 12 C-Atome, derartige Arylreste z.B. 6 bis 10 C-Atome und derartige Alkinylreste z.B. 2 bis 6 C-Atome enthalten. Als Heteroatome enthaltende Reste kommen z.B. Fluor-, Chlor-, Brom-, Iod-, Hydroxy-, $C_1$- bis $C_6$-Alkoxy-, $C_6$- bis $C_{10}$-Phenoxy-, Carboxy-, $C_1$- bis $C_6$-Alkoxycarbonyl-, Nitro-, Amid-, Nitril-, Mercapto-, Sulfonyl-, Phosphit- und Phosphatgruppen in Frage. Cycloalkylreste, Arylreste und Alkinylreste, insbesondere gesättigte Cycloalkylreste und Arylreste, können gegebenenfalls ihrerseits nochmals Substituenten aufweisen, beispielsweise $C_1$- bis $C_6$-Alkyl-, Fluor-, Chlor-, Brom-, Hydroxy-, $C_1$- bis $C_6$-Alkoxy-, Carboxy-, $C_1$- bis $C_6$-Alkoxycarbonyl-, Nitro-, Sulfonyl- und/oder Nitrilgruppen.

Soweit $R_1$, $R_2$ und/oder $R_3$ für gegebenenfalls substituiertes gesättigtes Cycloalkyl stehen, kommen hierfür beispielsweise 3 bis 12 C-Atome im Ringsystem und insgesamt, d.h. einschließlich gegebenenfalls vorhandener Substituenten, beispielsweise 3 bis 15 C-Atome enthaltende Cycloalkylreste in Frage. Als Substituenten an solchen gesättigten Cycloalkylresten kommen beispielsweise in Frage: gesättigte Alkylreste und/oder Heteroatome, wie Halogen-, Sauerstoff-, Schwefel- und/oder Stickstoff-atome enthaltende Reste. Derartige gesättigte Alkylreste können geradkettig oder verzweigt sein und beispielsweise 1 bis 6 C-Atome enthalten. Derartige Heteroatome enthaltende Reste können beispielsweise Fluor-, Chlor-, Brom-, Iod-, Hydroxy-, $C_1$—$C_6$-Alkoxy-, Carboxy-, $C_1$- bis $C_6$-Alkoxycarbonyl-, Nitro-, Nitril- und/oder Sulfonylreste sein.

Soweit $R_1$, $R_2$ und/oder $R_3$ für gegebenenfalls substituiertes Alkinyl stehen kommen beispielsweise Alkinylreste mit 1 bis 6 C-Atomen in Frage, die beispielsweise eine oder zwei Dreifachbindungen enthalten, wie Ethinyl, Propargyl, 1-Butinyl und 2-Butinyl. Als Substituenten kommen hier beispielsweise Fluor-, Chlor-, Brom- und/oder $C_1$- bis $C_6$-Alkylgruppen in Frage.

Soweit $R_1$ und $R_2$ gemeinsam mit dem C-Atom, an das sie gebunden sind, einen gegebenenfalls substituierten gesättigten Cycloalkylrest bilden kann es sich dabei beispielsweise um einen 3 bis 12 C-Atome im Ringsystem enthaltenden Cycloalkylrest handeln, der gegebenenfalls mit gesättigten, geradkettigen oder verzweigten Alkylresten mit z.B. 1 bis 6 C-Atomen und/oder mit Heteroatome, wie Halogen-, Sauerstoff-, Schwefel- und/oder Stickstoffatome, enthaltenden Resten substituiert sein kann.

Vorzugsweise sind $R_1$ und $R_2$ gleich oder verschieden und stehen jeweils für einen gesättigten, unsubstituierten, geradkettigen oder verzweigten Alkylrest mit 1 bis 6 C-Atomen, beispielsweise für Methyl, Ethyl, n-Propyl, i-Propyl, n-Butyl, sek.-Butyl, tert.-Butyl, n-Pentyl, Amyl-, i-Amyl- oder n-Hexyl.

Besonders bevorzugt stehen $R_1$ und $R_2$ für Methyl.

Vorzugsweise steht $R_3$ für einen gesättigten, unsubstituierten, geradkettigen oder verzweigten Alkylrest mit 1 bis 6 C-Atomen, für einen mit einer Hydroxygruppe oder einer gegebenenfalls durch Chlor substituierten Phenylgruppe substituierten geradkettigen oder verzweigten Alkylrest mit 1 bis 6 C-Atomen oder für einen unsubstituierten Alkinylrest, der 1 bis 6 C-Atome und eine Dreifachbindung enthält.

Besonders bevorzugt steht $R_3$ für Methyl, Ethyl, Ethinyl, Hydroxymethylen oder p-Chlorbenzyl.

Soweit $R_4$ und/oder $R_5$ für gegebenenfalls substituiertes gesättigtes Alkyl und gegebenenfalls substituiertes gesättigtes Cycloalkyl stehen kommen beispielsweise die Reste und Substituenten in Frage, die vorstehend bei der Erläuterung von $R_1$, $R_2$ und $R_3$ für solche Typen von Resten angegeben sind. Soweit $R_4$ und/oder $R_5$ für gegebenenfalls substituiertes gesättigtes Alkoxy und gegebenenfalls substituiertes gesättigtes Cycloalkoxy stehen kommen beispielsweise als Alkyl- und Cycloalkylteile dieser Reste ebenfalls die Reste und Substituenten in Frage, die vorstehend bei der Erörterung von $R_1$, $R_2$ und $R_3$ für solche Typen von Resten angegeben sind.

Wenn beide Reste $R_4$ und $R_5$ eine von Wasserstoff verschiedene Bedeutung haben befinden sich diese Reste vorzugsweise in den 3- und 4-Positionen.

Vorzugsweise steht $R_4$ für Wasserstoff und $R_5$ für einen der zuvor beschriebenen Reste, der sich in 3- oder 4-Position befindet.

Besonders bevorzugt steht $R_5$ für einen geradkettigen oder verzweigten, gesättigten $C_1$- bis $C_6$-Alkylrest oder für einen geradkettigen oder verzweigten gesättigten $C_1$- bis $C_6$-Alkoxyrest, beispielsweise für Methyl, Methoxy, Ethyl, Ethoxy, n-Propyl, n-Propoxy, i-Propyl, i-Propoxy, n-Butyl, n-Butoxy, sek.-Butyl, sek.-Butoxy, tert.-Butyl, tert.-Butoxy, n-Hexyl oder n-Hexoxy jeweils in 3- oder 4-Position.

Ganz besonders bevorzugt steht $R_5$ für Methyl oder Methoxy in 4-Position.

Die Arylaldimine der Formel (I) sind gut zugängliche Verbindungen, die auf an sich bekannte Weise (siehe z.B. W. D. Emmons und A. S. Pagano, Organic Synthesis, Coll. Vol. V, S. 191) durch Kondensation eines Amins der Formel (II)

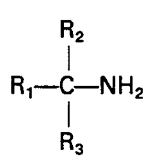

(II),

in der $R_1$, $R_2$ und $R_3$ die bei Formel (I) angegebene Bedeutung haben mit einem aromatischen Aldehyd der Formel (III)

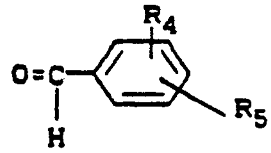

(III),

in der $R_4$ und $R_5$ die bei Formel (I) angegebene Bedeutung haben, erhalten werden können.

Als Lösungsmittel können im erfindungsgemäßen Verfahren z.B. die verschiedensten unsubstituierten und substituierten Kohlenwasserstoffs verwendet werden, die unter Reaktionsbedingungen flüssig sind und unerwünschte Nebenreaktionen nicht oder nur in ganz untergeordnetem Maße eingehen. Es können beispielsweise verwendet werden: aliphatische und cycloaliphatische Kohlenwasserstoffe mit 6 bis 12 C-Atomen, wie Hexan, Heptan, Octan, 2-Ethyl-hexan, Decan, Dodecan, Cyclohexan, Methylcyclopentan und Petrolether, weiterhin aromatische Kohlenwasserstoffe, wie Benzol, Nitrobenzol, Toluol, Ethylbenzol, Cumol, Diisopropylbenzol, Xylol und Chlorbenzol, weiterhin Sauerstoffhaltige Kohlenwasserstoffe wie Ether und Ester, z.B. Diethylether, Diisopropylether, Dibutylether, Tetrahydrofuran, Dioxan, Ethylenglykoldimethylether, Essigsäureethylester, Essigsäuremethylester, Essigsäurepropylester, Essigsäurebutylester, Propionsäureethylester, weiterhin chlorierte Kohlenwasserstoffe, wie Methylenchlorid, Chloroform, Tetrachlorkohlenstoff, 1-Chlorethan, 1,2-Dichlorethan, 1,1-Dichlorethan, 1,1,2,2-Tetrachlorethan, 1-Chlorpropan, 2-Chlorpropan, 1,3-Dichlorpropan, 2,3-Dichlorpropan, 1,2,3-Trichlorpropan, 1,1,2,3-Tetrachlorpropan, Butylchlorid, 1,2-Dichlorbutan, 1,4-Dichlorbutan, 2,3-Dichlorbutan, 1,3-Dichlorbutan, 1,2,3,4-Tetrachlorbutan, tert.-Butylchlorid, Amylchlorid, 1,2-Dichlorpentan, 1,5-Dichlorpentan, 1,2,3,4-Tetrachlorpentan, Cyclopentylchlorid, 1,2-Dichlorcyclopentylchlorid, Hexylchlorid, 1,2-Dichlorhexan, 1,6-Dichlorhexan, 1,2,3,4-Tetrachlorhexan, 1,2,5,6-Tetrachlorhexan, Cyclohexylchlorid, Chlorbenzol, Heptylchlorid, 1,2-Dichlorheptan, 1,2,3,4-Tetrachlorheptan, Cycloheptylchlorid, Octylchlorid, 1,2-Dichloroctan, 1,2,3,4-Tetrachloroctan und Cyclooctylchlorid.

Besonders bevorzugte Lösungsmittel sind von den chlorierten Kohlenwasserstoffen 1,2-Dichlorpropan und Tetrachlorkohlenstoff, von den aromatischen Kohlenwasserstoffen Benzol und Chlorbenzol, von den aliphatischen und cycloaliphatischen Kohlenwasserstoffen Cyclohexan und von den sauerstoffhaltigen Kohlenwasserstoffen Propionsäureethylester und Ethylenglykoldimethylester.

Verwendet werden können auch Lösungsmittelgemische der verschiedenen oben angegebenen Lösungsmittel.

Perpropionsäure gelöst in einem der genannten organischen Lösungsmittel kann z.B. nach dem in der DE—OS 2 262 970 beschriebenen Verfahren hergestellt werden, bei dem wäßriges Wasserstoffperoxid mit Propionsäure in Gegenwart von Schwefelsäure umgesetzt und anschließend die entstandene Perpropionsäure mit dem Lösungsmittel aus dem Reaktionsgemisch extrahiert wird. Gegebenenfalls kann die so erhaltene Perpropionsäurelösung in dem Lösungsmittel noch weiter gereinigt werden, insbesondere um den Gehalt an Wasser, Wasserstoffperoxid und/oder Schwefelsäure zu erniedrigen.

Im allgemeinen verwendet man die Perpropionsäure in Form einer Lösung in einem organischen Lösungsmittel. Derartige Perpropionsäurelösungen können beispielsweise 10 bis 30 Gew.-% Perpropionsäure, bezogen auf die Lösung enthalten. Die Arylaldimine der Formel (I) können als solche oder ebenfalls gelöst in einem oder mehreren der vorstehend genannten Lösungsmittel eingesetzt werden, wobei beliebig konzentrierte Lösungen zum Einsatz gelangen können. Das Molverhältnis von Perpropionsäure zu Arylaldimin der Formel (I) kann in weiten Grenzen schwanken. Beispielsweise kann dieses Molverhältnis 0,1:1 bis 10:1 betragen. Bevorzugt wird ein Molverhältnis von 0,5:1 bis 5:1 angewendet. Ganz besonders vorteilhaft ist es, ein Molverhältnis von 0,8 bis 1,2 Mol Perpropionsäure je Mol Arylaldimin der Formel (I) anzuwenden.

Der Wassergehalt der verwendeten Perpropionsäure soll im allgemeinen möglichst niedrig sein. Geeignet ist beispielsweise eine Perpropionsäurelösung mit einem Wassergehalt von bis zu 2 Gew.-%. Vorzugsweise verwendet man eine Perpropionsäurelösung, die weniger als 1 Gew.-% Wasser enthält. Besonders bevorzugt ist ein Wassergehalt von weniger als 0,1 Gew.-%.

Der Wasserstoffperoxidgehalt der verwendeten Perpropionsäure soll im allgemeinen ebenfalls möglichst niedrig sein. Er kann beispielsweise bis zu 1 Gew.-%, bezogen auf die Perpropionsäurelösung, betragen. Vorteilhaft arbeitet man mit einem Gehalt von weniger als 0,5 Gew.-% besonders vorteilhaft mit einer Perpropionsäurelösung, die einen Wasserstoffperoxidgehalt unterhalb 0,2 Gew.-% aufweist.

Der Mineralsäuregehalt der verwendeten Perpropionsäure soll ebenfalls möglichst niedrig sein. Vorteilhaft ist es, die erfindungsgemäße Umsetzung mit einer Perpropionsäurelösung durchzuführen, die einen Mineralsäuregehalt unterhalb von 50 ppm besitzt. Besonders vorteilhaft ist ein Mineralsäuregehalt von weniger als 10 ppm.

Die erfindungsgemäße Umsetzung mit Perpropionsäure kann beispielsweise bei Temperaturen im Bereich von 0 bis 100°C durchgeführt werden. Bevorzugt arbeitet man hier bei man 20 bis 80°C, besonders bevorzugt bei 25 bis 40°C. In Sonderfällen können die angegebenen Temperaturen auch unter- oder überschritten werden.

Neben der Arbeitsweise unter isothermen Bedingungen, d.h. Einhaltung einer einheitlichen Temperatur im gesamten Reaktionsgemisch, kann man die erfindungsgemäße Umsetzung mit

Perpropionsäure auch unter Ausbildung eines sogenannten Temperaturgradienten durchführen, der im allgemeinen mit fortschreitender Reaktion zunimmt. Man kann aber auch die Reaktion so führen, daß sich mit dem fortschreiten der Reaktion ein Gradient fallender Temperatur ausbildet.

Bei der praktischen Durchführung der erfindungsgemäßen Umsetzung mit Perpropionsäure wird vorzugsweise das Aryl aldimin der Formel (I) vorgelegt und dann die Perpropionsäure in Form einer Lösung in einem Lösungsmittel zugegeben. Die Reaktionstemperatur kann dabei vor oder nach der Zugabe der Perpropionsäure eingestellt werden. Man kann auch die beiden Komponenten bei Raumtemperatur gleichzeitig in das Reaktionsgefäß eingeben und dann die Reaktionstemperatur einstellen.

Die nach Durchführung der erfindungsgemäßen Umsetzung mit Perpropionsäure vorliegenden Reaktionsgemische enthalten im allgemeinen das verwendete organische Lösungsmittel, aus der Perpropionsäure entstandene Propionsäure, das gebildete Oxaziridin, sowie gegebenenfalls unumgesetztes Arylaldimin der Formel (I) und/oder Nebenprodukte.

Zur Durchführung der erfindungsgemäßen Hydrolyse ist es nicht unbedingt erforderlich, das gebildete Oxaziridin aus dem Reaktionsgemisch zu isolieren. Es ist lediglich erforderlich aus dem Reaktionsgemisch Propionsäure zu entfernen. Im allgemeinen ist es vorteilhaft, die Propionsäure zum größten Teil oder weitgehend vollständig zu entfernen, beispielsweise 80 bis 100 Gew.-% der vorhandenen Propionsäure. Im allgemeinen stören geringe Restmengen von Propionsäure das weitere Verfahren nicht oder nicht wesentlich. Beispielsweise können, ohne Nachteile in Kauf nehmen zu müssen, im allgemeinen bis zu 5 Gew.-% der nach der Umsetzung mit Perpropionsäure im Reaktionsgemisch vorliegenden Propionsäure dort verbleiben. Selbstverständlich kann das gebildete Oxaziridin auch isoliert werden, beispielsweise durch Destillation.

Die Aufarbeitung des nach der Umsetzung mit Perpropionsäure vorliegenden Reaktionsgemisches kann z.B. durch Destillation erfolgen. Dabei kann man so verfahren, daß man entweder nur Propionsäure oder Lösungsmittel und Propionsäure oder alle verdampfbaren Komponenten in der Reihenfolge ihrer Siedepunkte abdestilliert. Dabei ist es von Vorteil, die Destillation im Vakuum und unter Verwendung von Verdampfern, die kurze Verweilzeiten und eine Verdampfung mit geringer thermischer Belastung ermöglichen, durchzuführen.

Es ist weiterhin von Vorteil, vor einer solchen destillativen Aufarbeitung aus dem Reaktionsgemisch Propionsäure durch Extraktion, z.B. mit Wasser oder einer wäßrigalkalischen Lösung, zu entfernen. Aus der so erhaltenen wäßrigen Propionsäure- bzw. Alkalipropionatlösung kann die Propionsäure wieder, gegebenenfalls nach Ansäuern mit einer starken Säure, durch Extraktion mit einem geeigneten Lösungsmittel zurückgewonnen und gegebenenfalls zur Herstellung der Perpropionsäure verwendet werden. Wenn aus dem Reaktionsgemisch nur Propionsäure entfernt werden soll, kann dies auch durch eine solche Extraktion geschehen.

Man erhält so ein Produkt, das im allgemeinen im wesentlichen aus dem gebildeten Oxaziridin und dem dazu isomeren Nitron besteht und das gegebenenfalls noch organisches Lösungsmittel und gegebenenfalls restliche Propionsäure enthält. Das Verhältnis der beiden isomeren Verbindungen ist von den Reaktionsbedingungen, wie Temperatur und Verweilzeit, der thermischen Belastung bei der Aufarbeitung und vom eingesetzten Arylaldimin abhängig. Dieses Produkt kann ohne weitere Isolierung dieser Verbindungen und/oder gegebenenfalls weiterer noch vorhandener Verbindungen oder Lösungsmittel, zu der erfindungsgemäßen Hydrolyse eingesetzt werden.

Es ist ein wesentliches Merkmal des erfindungsgemäßen Verfahrens, daß diese Hydrolyse mit wäßriger Salzsäure durchgeführt wird. Dabei fallen die entsprechenden Hydroxylammoniumchloride in sehr guten Ausbeuten als kristalline, gut handhabbare Verbindungen an. Diese Hydrolyse kann bei verschiedenen Temperaturen erfolgen, beispielsweise bei 0 bis 100°C. Da diese Hydrolyse bei tiefen Temperaturen langsamer verläuft als bei höheren Temperaturen arbeitet man bevorzugt bei 20 bis 80°C, insbesondere bei 30 bis 60°C. Es kann im Einzelfall auch vorteilhaft sein, die angegebenen Temperatur zu über- oder unterschreiten. Das gilt insbesondere dann, wenn die Hydrolyse neben Wasser in Gegenwart eines oder mehrerer organischer Lösungsmittel durchgeführt wird.

Die wäßrige Salzsäure, die zu dieser Hydrolyse verwendet wird, kann sehr unterschiedliche Konzentrationen aufweisen. Geeignet sind beispielsweise Konzentrationen zwischen 1 und 37 Gew.-%. Bevorzugt sind Konzentrationen zwischen 3 und 37 Gew.-%, insbesondere solche zwischen 5 und 37 Gew.-%. Die Menge der Salzsäure kann beispielsweise 5 bis 200 Gew.-%, bezogen auf das vorliegende Oxaziridin, betragen. Vorzugsweise beträgt diese Menge von 30 bis 120 Gew.-%.

Die Hydrolyse kann auch in zusätzlicher Gegenwart von Lösungsmitteln durchgeführt werden. Geeignet sind hierfür insbesondere solche Lösungsmittel, in denen das Oxaziridin löslich ist und die mit Wasser mischbar sind. Selbstverständlich soll das Lösungsmittel gegenüber den angewendeten Reaktionsbedingungen stabil sein. Geeignet sind z.B. mit Wasser mischbare Ether, Alkohole, Ketone und Ester, wie Tetrahydrofuran, Dioxan, Ethylenglykoldimethylether, Methanol, Ethanol, Ethylenglykol, Aceton, Ameisensäuremethylester, Ethylenglykolmethylether-monoacetat und Essigsäuremethylester. Bevorzugt sind Dioxan, Ethylenglykoldimethylether, Methanol und Ethanol. Besonders bevorzugt ist Methanol.

Die Reaktionszeit für die Hydrolyse kann in weiten Grenzen schwanken. Im allgemeinen ist eine Reaktionszeit zwischen 30 und 100 Minuten ausreichend.

Neben dem gewünschten Hydroxylammoniumchlorid entsteht bei der Hydrolyse der aromatische Aldehyd der Formel (III), von dem sich das eingesetzte Arylaldimin der Formel (I) ableitet. Dieser Aldehyd

kann, falls gewünscht, aus der Hydrolysemischung, gegebenenfalls nach weiterem Zusatz von Wasser, mit einem organischen Lösungsmittel extrahiert werden, das zur Hydrolysemischung eine zweite Phase zu bilden vermag. Geeignete Extraktionsmittel sind beispielsweise Diethylether, Methylenchlorid, Chloroform, Essigester, Tetrachlorkohlenstoff, Methylisobutylketon, Benzol und Toluol. Aus dieser Lösungsmittelphase kann der Aldehyd durch Abziehen des Lösungsmittels zurückgewonnen und gegebenenfalls in die Herstellung der Verbindung der Formel (I) zurückgeführt werden.

Nach dem Abtrennen der organischen Phase verbleibt dann eine wäßrige Phase, in der das gewünschte Hydroxylammoniumchlorid gelöst ist. Das gewünschte Produkt kann im allgemeinen am einfachsten durch Einengen zur Kristallisation gebracht und dann z.B. durch Filtration oder Abdampfung aller flüchtigen Bestandteile isoliert werden.

Wenn der gebildete aromatische Aldehyd der Formel (III) nicht durch Extraktion aus dem Hydrolysegemisch abgetrennt wird, kann im allgemeinen aus diesem auch direkt das gewünschte Hydroxylammoniumchlorid auf entsprechende Weise isoliert werden.

Das erfindungsgemäße Verfahren gestattet die Herstellung von Hydroxylammoniumchloriden der Formel (IV)

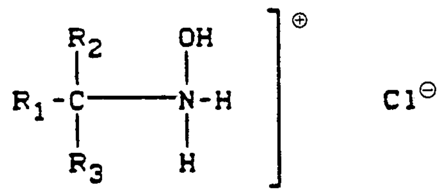

(IV),

in der $R_1$, $R_2$ und $R_3$ die bei Formel (I) angegebene Bedeutung haben, in guten Ausbeuten und bei einfacher Handhabung. Überraschend ist insbesondere, daß die zur Hydrolyse eingesetzte Salzsäure, im Gegensatz zu dem Verfahren von W. D. Emmons, J.A.C.S. *79*, 5739—5754 (1957) nicht oxidiert wird und damit die im Gegensatz zu den entsprechenden Hydroxylammoniumsulfaten bzw. -hydrogensulfaten gut kristallisierenden Hydroxylammoniumchloride der Formel (IV) gut hergestellt und isoliert werden können. Die verwendete Perpropionsäure hat den Vorteil, daß sie als Lösung in einem organischen Lösungsmittel eingesetzt werden kann und dann erheblich weniger sicherheitstechnischer Aufwand nötig ist als bei dem bekannten Verfahren, bei dem Peressigsäure in situ aus hochprozentigem Wasserstoffperoxid hergestellt wird.

Die Hydroxylammoniumchloride der Formel (IV) sind lagerstabile Verbindungen, aus denen die entsprechenden, weniger lagerstabilen Hydroxylamine gegebenenfalls durch eine einfache Behandlung mit Basen freigesetzt werden können.

Die folgenden Beispiele erläutern die vorliegende Erfindung ohne sie in irgendeiner Weise zu beschränken.

## BEISPIELE

### Beispiel 1

181 g (0,95 Mol) N-tert.-Butyl-p-methoxyphenylimin wurden bei 25°C und unter Rühren mit 503 g einer Lösung von Perpropionsäure in Benzol versetzt, die 17 Gew.-% Perpropionsäure, weniger als 0,1 Gew.-% Wasser, weniger als 0,2 Gew.-% Wasserstoffperoxid und weniger als 10 ppm Mineralsäure enthielt. Die Zutropfzeit betrug 2 Stunden. Danach wurde noch 2 Stunden bei 25°C nachgerührt, anschließend das Reaktionsgemisch mit verdünnter Natronlauge gewaschen und eingeengt bis praktisch das gesamte vorhandene Benzol abgedampft war. Es hinterblieben 189 g eines Rückstandes, der das dem eingesetzten Imin entsprechende Oxaziridin und das dem eingesetzten Imin entsprechende Nitron im Gewichtsverhältnis von 81:19 enthielt. Das entspricht (für beide Oxidationsprodukte zusammen) einer Ausbeute von 96,4% der Theorie.

104,5 g (0,5 Mol) des so erhaltenen Rückstandes wurden in 500 ml Methanol gelöst. Unter Rühren wurden 100 ml wäßrige HCl (37 gew.-%ig) zugetropft, wobei darauf geachtet wurde, daß die Temperatur 40°C nicht überstieg. Nach 60 Minuten wurde mit Wasser verdünnt und mit Diethylether ausgeschüttelt. Die wäßrige Phase wurde eingedampft und im Ölpumpenvakuum getrocknet. Es wurden 57 g farbloses, kristallines tert.-Butylhydroxylammoniumchlorid vom Schmelzpunkt 175 bis 180°C erhalten, was 91% Ausbeute bezogen auf die eingesetzten Oxidationsprodukte (Oxaziridin + Nitron) entspricht.

Tabelle 1

| Beispiel Nr. | eingesetztes Arylaldimin der Formel (I) | erhaltenes Hydroxylammoniumchlorid der Formel (IV) | Schmelzpunkt | Ausbeute |
|---|---|---|---|---|
| 2 | $R_1$ = $R_2$ = $CH_3$, $R_3$ = $C_2H_5$, $R_4$ = H, $R_5$ = p-Methoxy | $R_1$ = $R_2$ = $CH_3$, $R_3$ = $C_2H_5$ | 108-111° C | 75 % |
| 3 | $R_1$ = $R_2$ = $CH_3$, $R_3$ = C≡CH, $R_4$ = H, $R_5$ = p-Methoxy | $R_1$ = $R_2$ = $CH_3$, $R_3$ = C≡CH *) | 101-103° C | 80 % |
| 4 | $R_1$ = $R_2$ = $CH_3$, $R_3$ = $CH_2OH$, $R_4$ = H, $R_5$ = p-Methoxy | $R_1$ = $R_2$ = $CH_3$, $R_3$ = $CH_2OH$ | 104-106° C | 57 % |
| 5 | $R_1$ = $R_2$ = $CH_3$, $R_3$ = $CH_2$-p-Chlorphenyl, $R_4$ = H, $R_5$ = p-Methoxy | $R_1$ = $R_2$ = $CH_3$, $R_3$ = $CH_2$-p-Chlorphenyl | 157-159° C | 64 % |
| 6 | $R_1$ = $R_2$ = $R_3$ = $CH_3$, $R_4$ = H, $R_5$ = Methyl | $R_1$ = $R_2$ = $R_3$ = $CH_3$ | 175-180° C | 71 % |

*) Das entspricht Formel (V) mit $R_6$ = $R_7$ = $CH_3$ und $R_8$ = C≡CH.

Beispiele 2 bis 6

Es wurde verfahren wie in Beispiel 1, jedoch wurden entsprechende molare Mengen anderer Arylaldimine eingesetzt. Die Ausgangsarylaldimine, die erhaltenen Hydroxylammoniumchloride und sonstige Einzelheiten sind aus Tabelle 1 ersichtlich.

**Patentansprüche**

1. Verfahren zur Herstellung von N-Alkyl-substituierten Hydroxylammoniumchloriden, dadurch gekennzeichnet, daß man ein Arylaldimin der Formel

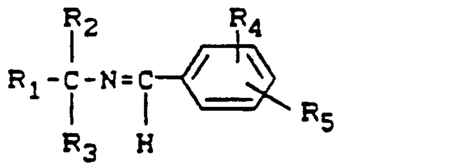

(I),

in der

$R_1$, $R_2$ und $R_3$ unabhängig voneinander für Wasserstoff, gegebenenfalls substituiertes gesättigtes Alkyl, gegebenenfalls substituiertes gesättigtes Cycloalkyl und/oder gegebenenfalls substituiertes Alkinyl stehen, wobei $R_1$ und $R_2$ gemeinsam mit dem C-Atom, an das sie gebunden sind, auch einen gegebenenfalls substituierten gesättigten Cycloalkylrest bilden können und

$R_4$ und $R_5$ unabhängig voneinander für Wasserstoff, gegebenenfalls substituiertes, gesättigtes Alkyl, gegebenenfalls substituiertes gesättigtes Cycloalkyl, gegebenenfalls substituiertes gesättigtes Alkoxy und/oder gegebenenfalls substituiertes gesättigtes Cycloalkoxy stehen, wobei mindestens einer der Reste $R_4$ und $R_5$ eine andere Bedeutung als Wasserstoff hat,

mit Perpropionsäure in Gegenwart eines Lösungsmittels umsetzt, dann zumindest Propionsäure abtrennt und anschließend eine Hydrolyse mit wäßriger Salzsäure durchführt.

2. Verfahren nach Anspruch 1, dadurch gekennzeichnet, daß man ein Arylaldimin der Formel (I) einsetzt, in der $R_1$ und $R_2$ gleich oder verschieden sind und jeweils für einen gesättigten, unsubstituierten, geradkettigen oder verzweigten Alkylrest mit 1 bis 6 C-Atomen stehen, $R_3$ für einen gesättigten, unsubstituierten, geradkettigen oder verzweigten Alkylrest mit 1 bis 6 C-Atomen, für einen mit einer Hydroxygruppe oder einer gegebenenfalls durch Chlor substituierten Phenylgruppe substituierten geradkettigen oder verzweigten Alkylrest mit 1 bis 6 C-Atomen oder für einen unsubstituierten Alkinylrest, der 1 bis 6 C-Atome und eine Dreifachbindung enthält, steht, $R_4$ für Wasserstoff steht und $R_5$ für einen geradkettigen oder verzweigten, gesättigten $C_1$- bis $C_6$-Alkylrest oder für einen geradkettigen oder verzweigten gesättigten $C_1$- bis $C_6$-Alkoxyrest in 3- oder 4-Position steht.

3. Verfahren nach Ansprüchen 1 und 2, dadurch gekennzeichnet, daß man die Perpropionsäure in Form einer Lösung in einem organischen Lösungsmittel einsetzt.

4. Verfahren nach Anspruch 3, dadurch gekennzeichnet, daß die Perpropionsäurelösung 10 bis 30 Gew.-% Perpropionsäure, weniger als 2 Gew.-% Wasser, weniger als 1 Gew.-% Wasserstoffperoxid und weniger als 50 ppm Mineralsäure enthält.

5. Verfahren nach Ansprüchen 1 bis 4, dadurch gekennzeichnet, daß man aus dem nach der Umsetzung mit Perpropionsäure vorliegenden Gemisch nur die Propionsäure entfernt.

6. Verfahren nach Ansprüchen 1 bis 5, dadurch gekennzeichnet, daß man die Umsetzung mit Perpropionsäure und die Hydrolyse mit Salzsäure unabhängig voneinander bei 0 bis 100°C durchführt.

7. Verfahren nach Ansprüchen 1 bis 6, dadurch gekennzeichnet, daß man die Hydrolyse in Gegenwart eines Lösungsmittels durchführt, in dem das jeweils auftretende Oxaziridin löslich und das mit Wasser mischbar ist.

8. Verfahren nach Ansprüchen 1 bis 7, dadurch gekennzeichnet, daß man die Hydrolyse während einer Reaktionszeit von 30 bis 100 Minuten durchführt.

**Revendications**

1. Procédé de production de chlorures d'hydroxylammonium N-alkyl-substitués, caractérisé en ce que l'on fait réagir une arylaldimine de la formule

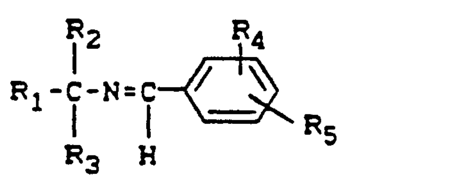

(I),

dans laquelle

$R_1$, $R_2$ et $R_3$ représentent indépendamment l'un de l'autre de l'hydrogène, un radical alkyle saturé

éventuellement substitué, un radical cycloalkyle saturé éventuellement substitué et/ou un radical alcynyle éventuellement substitué tandis que $R_1$ et $R_2$ peuvent former ensemble, avec l'atome de carbone auquel ils sont liés, un radical cycloalkyle saturé éventuellement substitué, et dans laquelle

$R_4$ et $R_5$ représentent indépendamment l'un de l'autre de l'hydrogène, un radical alkyle saturé éventuellement substitué, un radical cycloalkyle saturé éventuellement substitué, un radical alcoxy saturé éventuellement substitué et/ou un radical cycloalcoxy saturé éventuellement substitué tandis qu'au moins l'un des radicaux $R_4$ et $R_5$ ne représente pas de l'hydrogène,

avec l'acide perpropionique en présence d'un solvant, que l'on sépare au moins l'acide propionique et que l'on effectue ensuite une hydrolyse avec de l'acide chlorhydrique aqueux.

2. Procédé selon la revendication 1, caractérisé en ce que l'on utilise une arylaldimine de la formule (I) dans laquelle $R_1$ et $R_2$ sont identiques ou différents et représentent chacun un radical alkyle à 1 à 6 atomes de carbone saturé et non substitué à chaîne droite ou ramifiée, $R_3$ représente un radical alkyle à 1 à 6 atomes de carbone saturé non substitué et à chaîne droite ou ramifiée, un radical alkyle à 1 à 6 atomes de carbone à chaîne droite ou ramifiée substitué par un groupe hydroxy ou un groupe phényle substitué éventuellement par du chlore, ou un radical alcynyle non substitué contenant de 1 à 6 atomes de carbone et une liaison triple, $R_4$ représente de l'hydrogène et $R_5$ un radical alkyle en $C_1$—$C_6$ saturé à chaîne droite ou ramifiée ou un radical alcoxy en $C_1$ à $C_6$ saturé et à chaîne droite ou ramifiée, en position 3 ou 4.

3. Procédé selon les revendications 1 et 2, caractérisé en ce que l'on utilise l'acide perpropionique sous forme d'une solution dans un solvant organique.

4. Procédé selon la revendication 3, caractérisé en ce que la solution d'acide perpropionique contient de 10 à 30% en poids d'acide perpropionique, moins de 2% en poids d'eau, moins de 1% en poids de peroxyde d'hydrogène et moins de 50 ppm d'acide minéral.

5. Procédé selon les revendications 1 à 4, caractérisé en ce que l'on élimine du mélange existant après la réaction avec l'acide propionique, uniquement l'acide propionique.

6. Procédé selon les revendications 1 à 5, caractérisé en ce que l'on effectue la réaction avec l'acide perpropionique et l'hydrolyse avec l'acide chlorhydrique indépendamment l'une de l'autre, à une température de 0 à 100°C.

7. Procédé selon les revendications 1 à 6, caractérisé en ce que l'on effectue l'hydrolyse en présence d'un solvant dans lequel l'oxaziridine obtenue est soluble et qui est miscible avec l'eau.

8. Procédé selon les revendications 1 à 7, caractérisé en ce que l'on effectue l'hydrolyse pendant une durée de réaction de 30 à 100 minutes.

**Claims**

1. Process for the preparation of N-alkyl-substituted hydroxylammonium chlorides, characterized in that an aryl-aldimine of the formula

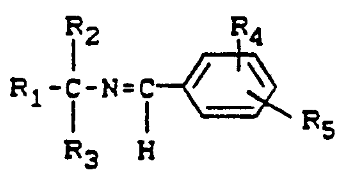

(I),

in which

$R_1$, $R_2$ and $R_3$, independently of one another, represent hydrogen, optionally substituted saturated alkyl, optionally substituted saturated cycloalkyl and/or optionally substituted alkinyl, and $R_1$ and $R_2$, together with the C atom to which they are bonded, can also form an optionally substituted saturated cycloalkyl radical, and

$R_4$ and $R_5$, independently of one another, represent hydrogen, optionally substituted saturated alkyl, optionally substituted saturated cyclo-alkyl, optionally substituted saturated alkoxy and/or optionally substituted saturated cyclo-alkoxy, at least one of the radicals $R_4$ and $R_5$ having a meaning other than hydrogen,

is reacted with perpropionic acid in the presence of a solvent, propionic acid at least is then separated off, and subsequently hydrolysing with aqueous hydrochloric acid.

2. Process according to Claim 1, characterized in that an arylaldimine of the formula (I) is employed in which $R_1$ and $R_2$ are identical or different and each represent a saturated, unsubstituted, straight-chain or branched alkyl radical with 1 to 6 C atoms, $R_3$ represents a saturated, unsubstituted, straight-chain or branched alkyl radical with 1 to 6 C atoms, a straight-chain or branched alkyl radical with 1 to 6 C atoms which is substituted by a hydroxyl group or by a phenyl group which is optionally substituted by chlorine, or an unsubstituted alkinyl radical which contains 1 to 6 C atoms and one triple bond, $R_4$ represents hydrogen and $R_5$ represents a straight-chain or branched, saturated $C_1$- to $C_6$-alkyl radical or a straight-chain or branched saturated $C_1$- to $C_6$-alkoxy radical in the 3- or 4-position.

3. Process according to Claims 1 and 2, characterized in that the perpropionic acid is employed in the form of a solution in an organic solvent.

4. Process according to Claim 3, characterized in that the perpropionic acid solution contains 10 to 30% by weight of perpropionic acid, less than 2% by weight of water, less than 1% by weight of hydrogen peroxide and less than 50 ppm of mineral acid.

5. Process according to Claims 1 to 4, characterized in that only the propionic acid is removed from the mixture present after the reaction with perpropionic acid.

6. Process according to Claims 1 to 5, characterized in that the reaction with perpropionic acid and the hydrolysis with hydrochloric acid are carried out independently of one another at 0 to 100°C.

7. Process according to Claims 1 to 6, characterized in that the hydrolysis is carried out in the presence of a solvent in which the oxaziridine occurring in each case is soluble and which is miscible with water.

8. Process according to Claims 1 to 7, characterized in that the hydrolysis is carried out during a reaction time of 30 to 100 minutes.